# EUROPEAN PATENT APPLICATION

(11) **EP 2 803 973 A1**
(43) Date of publication of application: **19.11.2014**
(21) Application number: 12788412.0
(22) Date of filing: 23.07.2012
(51) Int. Cl.: G01N 21/17

(54) **OPTICAL PROBE AND OPTICAL MEASUREMENT METHOD**

(30) Priority: 15.02.2012 JP 2012030956
(71) Applicant: Sumitomo Electric Industries, Ltd., Chuo-ku Osaka-shi Osaka 541-0041 (JP)
(72) Inventor: HASEGAWA Takemi, Yokohama-shi Kanagawa 244-8588 (JP); HIRANO Mitsuharu, Yokohama-shi Kanagawa 244-8588 (JP); TANAKA Masato, Yokohama-shi Kanagawa 244-8588 (JP)
(86) International application number: PCT/JP2012/068605
(87) International publication number: WO 2013/121602

(57) **Abstract**

An optical measurement method suitable for measuring a distribution of lipid in a blood vessel and an optical probe suitable for use in such a method are provided. An optical probe 10 includes an optical fiber 11 for transmitting light between a proximal end 11a and a distal end 11b, an optical connecter 12 being connected to the optical fiber 11 at the proximal end 11 a, a focusing optical system 13 and a deflection optical system 14 each being connected to the optical fiber 11 at the distal end 11b, a support tube 15 and a jacket tube 16 each surrounding the optical fiber 11 to extend along the optical fiber 11, and a buffer fluid 17 filled in the inner lumen of the jacket tube. The optical fiber 11 has a cutoff wavelength shorter than 1.53 µm. The optical fiber 11, the focusing optical system 13, the deflection optical system 14, and the buffer fluid 17 and jacket tube 16 on an optical path coupled to a fundamental mode of the optical fiber each have the light transmittance of -2 dB to 0 dB in a wavelength band of 1.6 µm to 1.8 µm.

## Description

### Technical Field

The present invention relates to an optical probe to be used in measurement through the use of an approach of Optical Coherence Tomography (OCT).

### Background Art

As the approach for measuring the tomographic structure of an inner lumen of a lumenal-shaped object such as a blood vessel, Optical Coherence Tomography (OCT) is known, and furthermore an optical probe inserted, for use, into the inner lumen of an object for this OCT measurement is also known (see Patent Literature 1). In the OCT measurement, a graded index optical fiber connected to the tip (distal end) of a single-mode optical fiber serves as a lens, and is configured such that the working distance thereof is longer than 1 mm and the spot size thereof is smaller than 100 µm, and thus an object having an internal radius larger than 1 mm can be optically measured with a spatial resolution finer than 100 µm.

The OCT measurement is used in diagnosing a lesion in a blood vessel and selecting a method of treatment. The OCT measurement of a lesion provides a tomographic image of the lesion. In the tomographic image, inside the lesion, a region that strongly scatters light is brightly displayed and a region that only weakly scatters light is displayed, as a monochromatic image with a dark gradation. Since the pattern of a bright and dark distribution of this image varies according to lesions, it is known that the type of the lesion can be estimated from the bright and dark pattern of the image to a certain extent (see Non-Patent Literature 1).

### Citation List

### Patent Literature

Patent Literature 1 US Patent No. 6,445,939
Patent Literature 2 US Patent Application No. 2002/0151823

### Non Patent Literature

Non Patent Literature 1 W. M. Suh, Circ Cardiovasc Imaging. 2011; 4: 169-178

### Summary of Invention

### Technical Problem

The present inventor has found that it may be difficult for an OCT device using a conventional optical probe, to identify the type of a lesion, e.g., it is difficult to distinguish between a lipid-rich plaque and a fibrocalcific plaque.

As described also in Non-Patent Literature 1, the lipid-rich plaque is characterized by a dark gradation and an diffuse contour, while the fibrocalcific plaque is characterized by a dark gradation and a sharp contour. However, since the brightness and darkness of gradation is relative, it is difficult to distinguish between the brightness and darkness if the variations due to an individual difference, a measurement condition, and the like are added. Moreover, it is often difficult to recognize the sharpness of a contour because an actual lesion has various shapes of patterns.

The present invention can provide an optical measurement method capable of dissolving the above-described problems and suitable for measuring a distribution of lipid in a blood vessel, and an optical probe suitable for use in such a method.

### Solution to Problem

An optical probe according to one aspect of the present invention can comprise: an optical fiber for transmitting light between a proximal end and a distal end; an optical connecter being connected to the optical fiber at the proximal end; a focusing optical system being connected to the optical fiber at the distal end and focusing light emitted from the distal end of the optical fiber; a deflection optical system being connected to the optical fiber at the distal end and deflecting light emitted from the distal end of the optical fiber; a jacket tube surrounding the optical fiber to extend along the optical fiber, and being rotatable relative to the optical fiber, the optical connecter, the focusing optical system, and the deflection optical system; and a buffer fluid filled in an inner lumen of the jacket tube. Furthermore, the optical fiber has a cutoff wavelength shorter than 1.53 µm, and the optical fiber, the focusing optical system, the deflection optical system, and the buffer fluid and jacket tube on an optical path coupled to a fundamental mode of the optical fiber have a light transmittance of -2 dB to 0 dB (between -2 dB and 0 dB) in a wavelength band of 1.6 µm to 1.8 µm (between 1.6 µm and 1.8 µm).

In the optical probe according to one aspect of the present invention, each of the optical fiber, the focusing optical system, and the deflection optical system comprises either silica glass or borosilicate glass, the buffer fluid is any one of a physiological saline solution, a dextran solution, and a silicone oil, the jacket tube comprises any one of FEP, PFA, PTFE, PET, and nylon, and a relative refractive index difference at one of an interface between the deflection optical system and the buffer fluid and an interface between the buffer fluid and the jacket tube can differ from an relative refractive index difference at the other interface, by 3.2 times or more.

An optical measurement method according to one aspect of the present invention, through the use of the optical probe according to claim 1, a light source generating light in a wavelength band of 1.6 µm to 1.8 µm (between 1.6 µm and 1.8 µm), an optical branching unit branching light emitted from the light source into two and outputting the resulting light as illumination light and reference light, an optical detector detecting light in the wavelength band, and an analyzer analyzing a light attenuation spectrum in the wavelength band and acquiring an analysis result obtained by the analysis, as image information, can comprise the steps of: irradiating an object with illumination light, the illumination light output from the optical branching unit to enter the proximal end of the optical fiber and to be emitted from the distal end; guiding back-reflection light to the optical detector, the back-reflection light generated by the object along with the irradiation, to enter the distal end of the optical fiber and to be emitted from the proximal end, while guiding the reference light output from the optical branching unit to the optical detector; detecting, with the optical detector, interference light caused by the back-reflection light and the reference light; and analyzing a spectrum of the back-reflection light with the analyzer, and acquiring distribution information of a substance inside the object as image information.

In an optical measurement method according to one aspect of the present invention, each of the optical fiber, the focusing optical system, and the deflection optical system comprises either silica glass or borosilicate glass, the buffer fluid is any one of a physiological saline solution, a dextran solution, and a silicone oil, the jacket tube comprises any one of FEP, PFA, PTFE, PET, and nylon, and a relative refractive index difference at one of an interface between the deflection optical system and the buffer fluid and an interface between the buffer fluid and the jacket tube can differ from a relative refractive index difference at the other interface, by 3.2 times or more.

The optical measurement method according to one aspect of the present invention can further comprise the steps of: extracting a spectral component having an absorption peak in a wavelength range of 1.70 to 1.75 µm in a spectrum of the back-reflection light, with the analyzer; and analyzing distribution information of lipid on the basis of the spectral component, and acquiring the analysis result as image information.

Moreover, the optical measurement method according to one aspect of the present invention may further comprise the steps of: detecting, with the optical detector, interference light caused by reflected light and the reference light, the reflected light caused by reflection of the illumination light output from the optical branching unit by the one of the interfaces and reaching the optical detector after the reflection; conducting Fourier analysis of a spectrum of the reflected light in a limited wavelength band and calculating an autocorrelation function as a function of delay time, with the analyzer; and calculating wavelength dependency that a delay time bringing the value of this autocorrelation function to a peak has among the wavelength bands, and calculating an estimated value of wavelength dispersion of the back-reflection light.

### Advantageous Effects of Invention

According to one aspect of the present invention, for example, a distribution of lipid in a blood vessel, which has been difficult to make a measurement by using the conventional art, can be measured.

### Brief Description of Drawings

FIG. 1 is a drawing showing the configuration of an OCT device 1 including an optical probe 10 of an embodiment of the present invention.
FIG. 2 is a drawing showing the spectrum of transmittance of each of a lipid-rich plaque, a normal blood vessel, and lard.

### Description of Embodiments

Hereinafter, with reference to the accompanying drawings, an embodiment of the present invention will be described in detail. In illustrating the drawings, the same reference numeral is attached to the same element to omit the repeated explanation thereof.

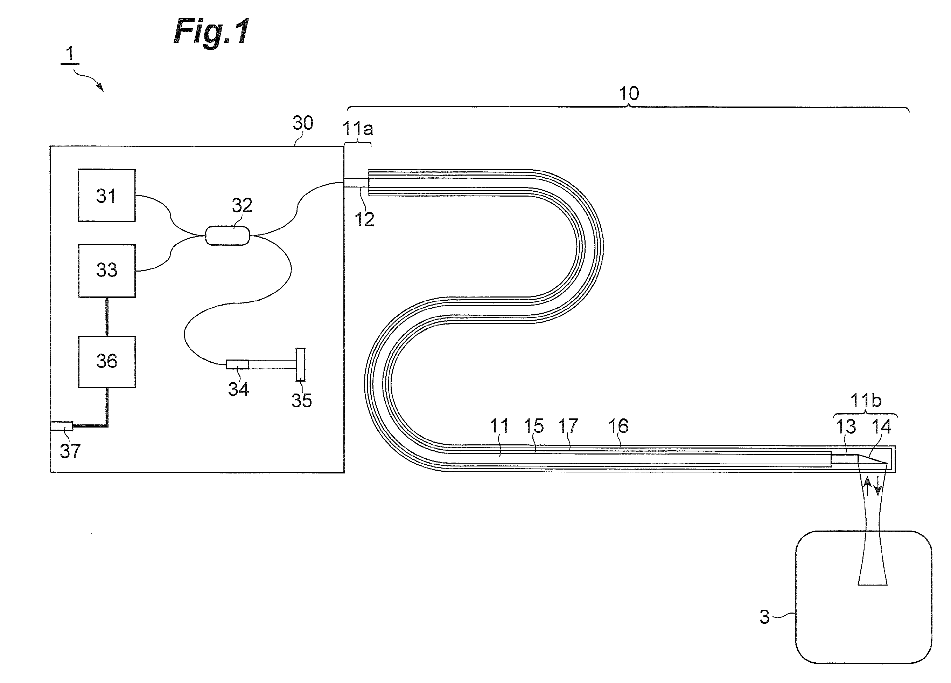

FIG. 1 is a drawing showing the configuration of an OCT device 1 including an optical probe 10 of an embodiment of the present invention. The OCT device 1 includes an optical probe 10 and a measurement unit 30, and acquires a light interference tomographic image of an object 3 with a method (optical measurement method) to be described below, through the use of the optical probe 10 and the measurement unit 30.

The optical probe 10 includes an optical fiber 11 for transmitting light between a proximal end 11 a and a distal end 11b, an optical connecter 12 being connected to the optical fiber 11 at the proximal end 11 a, a focusing optical system 13 and a deflection optical system 14 each optically being connected to the optical fiber 11 at the distal end 11b, a support tube 15 and a jacket tube 16 each surrounding the optical fiber 11 and extending along the optical fiber 11, and a buffer fluid 17 filled in an inner lumen of the jacket tube 16. The optical connecter 12 is optically connected to the measurement unit 30. The optical fiber 11 has a cutoff wavelength shorter than 1.53 µm. The optical fiber 11, the focusing optical system 13, the deflection optical system 14, and the buffer fluid 17 and jacket tube 16 on an optical path coupled to a fundamental mode of the optical fiber 11 each have the light transmittance of -2 dB to 0 dB (between -2 dB and 0 dB) in a wavelength band of 1.6 µm to 1.8 µm (between 1.6 µm and 1.8 µm).

The optical fiber 11 has a length of 1 to 2 m (between 1 m and 2 m) and is made up of silica glass. The optical fiber 11 may have, in a wavelength range of 1.6 µm to 1.8 µm (between 1.6 µm and 1.8 µm), a transmission loss of 2 dB or less, and may also have a transmission loss of 1 dB or less. The optical fiber 11 has a cutoff wavelength of 1.53 µm or less, and operates in a single-mode in the above-described wavelength range. As such an optical fiber, optical fibers compliant with ITU-TG.652, G.654, and G.657 can be utilized. The optical fiber compliant with ITU-TG.654A or ITU-TG.654C has a low transmission loss of 0. 22 dB/km or less at a wavelength of 1.55 µm, typically has a core of pure silica glass, has a low nonlinear optical coefficient, and can reduce the noise due to nonlinear optical effects, such as self-phase modulation.

At the distal end 11b of the optical fiber 11, a graded index (GRIN) lens as the focusing optical system 13 and a mirror as the deflection optical system 14 are serially fusion-spliced. The focusing optical system 13 collects the light emitted from the distal end 11b of the optical fiber 11. The deflection optical system 14 deflects the light emitted from the distal end 11b of the optical fiber 11 in radial direction.

The lens (focusing optical system 13) and the mirror (deflection optical system 14) are made up of either silica glass or borosilicate glass, and have transmission loss of 2 dB or less in the wavelength range of 1.6 µm to 1.8 µm (between 1.6 µm and 1.8 µm). The mirror has a structure, in which a flat reflective surface having an angle of 35 to 55 degrees (between 35 degrees and 55 degrees) relative to an axis is formed on cylindrical glass. This flat reflective surface, even if it is used as it is, can reflect light, but the vapor-deposition of either aluminum or gold onto the reflective surface can increase the reflectance at the wavelength of 1.6 to 1.8 µm (between 1.6 µm and 1.8 µm).

The optical fiber 11 is housed in the inner lumen of the support tube 15. The support tube 15 is fixed to at least a part of the optical fiber 11 and to the optical connecter 12. As a result, when the optical connecter 12 is rotated, the support tube 15 also rotates accordingly, and furthermore a rotary torque is transmitted to the optical fiber 11, and thus the optical fiber 11, the focusing optical system 13, the deflection optical system 14, and the support tube 15 integrally rotate. Therefore, as compared with the case where only the optical fiber 11 is rotated, a torque loaded on the optical fiber 11 is reduced, and fracturing of the optical fiber 11 due to the torque can be prevented.

The support tube 15 can have a thickness of 0.15 mm or more and also can have Young's modulus of 100 to 300 GPa (between 100 GPa and 300 GPa) comparable to that of stainless. The support tube 15 may not necessarily be circumferentially linked, and may be constructed by stranding approximately 5-20 lines, thereby being able to adjust the flexibility. Such a support tube is disclosed in Patent Literature 2.

The optical fiber 11, the focusing optical system 13, the deflection optical system 14, and the support tube 15 are housed in the inner lumen of the jacket tube 16, and can rotate therein. This prevents the rotating portion from making contact with the object 3 and damaging the object 3. The illumination light is emitted from the deflection optical system 14, transmits through the jacket tube 16, and the object 3 is irradiated with the illumination light. The jacket tube 16 is made up of any one of FEP, PFA, PTFE, PET, and nylon, has a thickness of 10 to 50 µm (between 10 µm and 50 µm), and has a transparency having a transmission loss of 2 dB or less at the wavelength of 1.6 to 1.8 µm (between 1.6 µm and 1.8 µm).

The inner lumen of the jacket tube 16 is filled with the buffer fluid 17. The buffer fluid 17 reduces friction between the outer surface of the rotating support tube 15 and the inner surface of the jacket tube 16, and also adjusts the variation of the refractive index in an optical path between the deflection optical system 14 and the jacket tube 16. The support tube 15 is rotatable relative to the focusing optical system 13 and the deflection optical system 14. The buffer fluid 17 is any one of physiological saline solution, dextran solution, and silicone oil, and has a transmission loss of 2 dB or less at the wavelength of 1.6 to 1.8 µm (between 1.6 µm and 1.8 µm).

The measurement unit 30 includes a light source 31 generating light, an optical branching unit 32 branching the light emitted from the light source 31 and outputting the resulting light as illumination light and reference light, an optical detector 33 detecting the light having reached from the optical branching unit 32, an optical terminal 34 for outputting the reference light having reached from the optical branching unit 32, a reflecting mirror 35 reflecting the reference light having been output from the optical terminal 34, to the optical terminal 34, an analyzer 36 analyzing a spectrum (light attenuation spectrum) of the light detected by the optical detector 33, and an output port 37 for outputting the result (image information) of the analysis made by the analyzer 36. The analyzer 36 acquires the analysis result (distribution information of a substance inside the object 3) obtained by the analyzer 36, as image information.

The light output from the light source 31 in the measurement unit 30 is branched by the optical branching unit 32 into two and is output as the illumination light and the reference light. The illumination light output from the optical branching unit 32 is incident upon the proximal end 11 a of the optical fiber 11 via the optical connecter 12, is guided by the optical fiber 11 and emitted from the distal end 11b, and the object 3 is irradiated with illumination light, via the focusing optical system 13 and the deflection optical system 14. The back-reflection light generated in response to the irradiation of the object 3 with the illumination light is incident upon the distal end 11b of the optical fiber 11 via the deflection optical system 14 and the focusing optical system 13, is guided by the optical fiber 11 and emitted from the proximal end 11a, and is coupled to the optical detector 33 via the optical connecter 12 and the optical branching unit 32.

The reference light output from the optical branching unit 32 is emitted from the optical terminal 34 and reflected by the reflecting mirror 35, and is coupled to the optical detector 33 via the optical terminal 34 and the optical branching unit 32. The back-reflection light from the object 3 and the reference light interfere with each other in the optical detector 33, and this interference light is detected by the optical detector 33. A spectrum of the interference light is input to the analyzer 36. In the analyzer 36, the spectrum of the interference light is analyzed and a distribution of back reflection efficiency at each point inside the object 3 is calculated. A tomographic image of the object 3 is calculated on the basis of this calculation result, and is output from the output port 37 as an image signal.

Note that the mechanism in which the illumination light emitted from the distal end 11b of the optical fiber 11 returns to the distal end 11b of the optical fiber 11 again via the object 3 includes, strictly speaking, reflection, refraction, and scattering. However, since these differences are not essential to the present embodiment, the reflection, refraction, and scattering are collectively referred to as back reflection in this specification, for simplification.

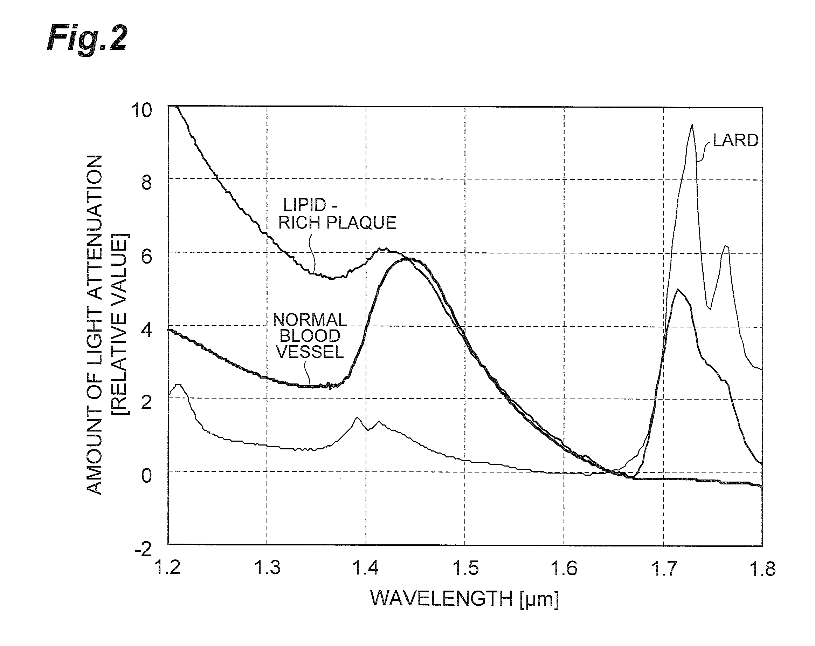

In the present embodiment, in the measurement unit 30, the light source 31 generates broad band light, the spectrum of which continuously spreads across the wavelength range of 1.6 µm to 1.8 µm (between 1.6 µm and 1.8 µm). In this wavelength range, as shown in FIG. 2, the lipid-rich plaque has an absorption peak at the wavelength of 1.70 to 1.75 µm (between 1.70 µm and 1.75 µm), and differs from normal blood vessels in this point. Since lard which is pure lipid also has a similar absorption peak, this absorption peak may be due to the contribution by lipid. Accordingly, when the object 3 including lipid is measured, the spectrum of interference light is affected by absorption due to lipid, and the spectrum shows a large attenuation at the wavelength of 1.70 to 1.75 µm (between 1.70 µm and 1.75 µm), as compared with the adjacent wavelength bands. Here, the analyzer 36 extracts the spectral component having an absorption peak at a wavelength range of 1.70 to 1.75 µm (a range between 1.70 µm and 1.75 µm) in the spectrum of the back-reflection light, analyzes distribution information of lipid on the basis of this spectral component, and acquires this analysis result as image information.

Furthermore, since the spectrum of interference light also has the information about a tomographic structure of the object 3, the information about the tomographic structure of the object 3 is obtained by selecting a wavelength band with a smaller influence of the absorption of a substance and conducting Fourier analysis of the spectrum. By analyzing the tomographic structure information and the lipid absorption information in combination, a tomographic image displaying the lipid in distribution can be calculated.

In this calculation, since both the absorption of lipid itself and the distribution of lipid affect the spectrum, a plurality of distributions of lipid can correspond to one spectrum. However, as described in Non-Patent Literature 1, it is for example known that lipid has characteristics of a scattering intensity lower than that of a normal blood vessel, and thus a distribution of lipid can be obtained by selecting a solution that most matches such knowledge.

Because all of the optical fiber 11, the focusing optical system 13, the deflection optical system 14, the buffer fluid 17, and the jacket tube 16 are not the same substance, the refractive indices are not necessarily equal to each other and light may reflect at the interface between each other. Since such reflected light generated at the interface of the optical probe 10 is mixed with the back-reflection light from the object 3 and detected, the reflected light may cause noise. However, in the present embodiment, the reflected light generated at the interface of the optical probe 10 is used for calibration of a measurement system.

In the OCT measurement, because the back-reflection light from the object 3 and the reference light goes through mutually different optical paths, the wavelength dispersion on the optical paths may differ from each other. If the wavelength dispersion differs, the group delay of light differs depending on the wavelength. It is known that in the OCT measurement, an autocorrelation function is calculated as a function of group delay time by conducting Fourier analysis of a spectrum, which is a function of wavelengths, and on the basis of this calculation result, a tomographic image is generated, and thus the spatial resolution of the tomographic image degrades if the group delay time differs depending on the wavelength. It is known that this problem can be resolved, by measuring, before measuring the object 3, a reference object such as a mirror, in place of the object 3 and measuring the influence of wavelength dispersion in advance, and carrying out data processing for compensating for the dispersion on the basis of the result.

However, in the present embodiment, since the spectrum information is used not only in capturing a tomographic image but also in estimation of a distribution of substances, the OCT of the present embodiment becomes more susceptible to the influence of wavelength dispersion as compared with the conventional OCT. Therefore, in the conventional method of performing the dispersion compensation before measuring the object 3, the variation in the wavelength dispersion caused by a mechanical variation and a temperature variation of the measurement system, which may occur during measurement, may affect the estimation of the distribution of substances. Therefore, it is possible to measure the reflection at the interface of the optical probe 10 at the distal end 11b during measurement, immediately before measurement, or immediately after measurement, and to perform the dispersion compensation processing.

Specifically, the reflected light generated at the interface of the optical probe 10 at the distal end 11b and the reference light are caused to interfere with each other, and the resulting light is detected by the optical detector 33. Then, the analyzer 36 conducts Fourier analysis of the wavelength spectrum in a plurality of limited wavelength bands and calculates an autocorrelation function, and further estimates a value of wavelength dispersion such that the position of a reflection peak on this autocorrelation function does not vary with the wavelength band used for analysis (in other words, the analyzer 36 calculates the wavelength dependency that the delay time bringing the value of the autocorrelation function to a peak has among wavelength bands, and calculates the estimated value of wavelength dispersion of the back-reflection light), and numerically adds a dispersion so as to cancel out the estimated wavelength dispersion, thereby being able to perform the dispersion compensation processing.

When reflected light with an intensity is generated, which is observable and does not saturate the optical detector 33 in one interface of the optical probe 10 at the distal end 11b, this purpose can be achieved. In the OCT measurement, the reflectance typically in a range of -100 to -50 dB (between -100 dB and -50 dB) can be measured. Then, in either one of an interface between the optical fiber 11 and the focusing optical system 13, an interface between the focusing optical system 13 and the deflection optical system 14, an interface between the deflection optical system 14 and the buffer fluid 17, an interface between the buffer fluid 17 and the jacket tube 16, and an interface between the jacket tube 16 and an external medium, reflection having a reflectance of -100 to -50 dB (between -100 dB and -50 dB) and having a reflectance higher than the other interfaces by 10 dB or more can be generated.

Here, the reflectance at the interface is a ratio of the power of light reflected by this interface and re-coupled to the core of the optical fiber 11, to the power of light emitted from the core of the optical fiber 11 at the distal end 11b and incident upon the interface. Accordingly, the reflectance at the interface depends not only on the variation of the refractive index at the interface but on the shape of the interface. Since any of an interface between the deflection optical system 14 and the buffer fluid 17, an interfaces between the buffer fluid 17 and the jacket tube 16, and an interface between the jacket tube 16 and the external medium is cylindrical, the reflectance deteriorates by approximately 0 to 30 dB due to the effect of their shapes. The external medium existing outside the jacket tube 16 is typically blood or physiological saline solution when the object 3 is a blood vessel, and the refractive index (the value at a wavelength of 589 nm which is a typical refractive-index evaluation wavelength, and the same is true hereinafter) is 1.33.

Then, one possible combination is as follows: the jacket tube 16 is made up of either FEP or PFA (refractive index of 1.34), the buffer fluid 17 is physiological saline solution (refractive index of 1.33), and the optical fiber 11, the focusing optical system 13, and the deflection optical system 14 is made up of silica glass. At that time, the relative refractive index difference at the interface between the optical fiber 11 and the focusing optical system 13 becomes 0%, the relative refractive index difference at the interface between the focusing optical system 13 and the deflection optical system 14 becomes 0%, the relative refractive index difference at the interface between the deflection optical system 14 and the buffer fluid 17 becomes 8.99%, the relative refractive index difference at the interface between the buffer fluid 17 and the jacket tube 16 becomes 0.82%, and the relative refractive index difference at the interface between the jacket tube 16 and the external medium becomes 0.82%. The relative refractive index difference at one of the interface between the deflection optical system 14 and the buffer fluid 17 and the interface between the buffer fluid 17 and the jacket tube 16 differs from the relative refractive index difference at the other interface, by 3.2 times or more. Note that, when the refractive indexes of the mediums on both sides of an interface are set to n1 and n2, the relative refractive index difference at the interface is defined as a formula: 2(n1-n2)/(n1+n2).

In this case, the relative refractive index difference of 8.99% at the interface between the deflection optical system 14 and the buffer fluid 17 is 11 times larger than that at other interfaces. Since the reflectance at an interface is proportional to the square of a relative refractive index difference, the reflectance at the interface between the deflection optical system 14 and the buffer fluid 17 is higher by 21 dB or more than the reflectance at other interfaces. Note that, since the respective refractive indices of the optical fiber 11, the focusing optical system 13, and the deflection optical system 14 coincide with each other, the reflectance at the interface between these can be neglected. As a result, the reflections at a plurality of interfaces do not overlap with each other on an OCT tomographic image, the reflection peak at the interface between the deflection optical system 14 and the buffer fluid 17 can be clearly observed, and thus the wavelength dispersion can be calibrated using this reflection peak.

### Industrial Applicability

An optical measurement method suitable for measuring a distribution of lipid in a blood vessel and an optical probe suitable for use in such a method can be provided.

### Reference Signs List

1... OCT device, 3... object, 10... optical probe, 11... optical fiber, 11 a... proximal end, 11b... distal end, 12... optical connecter, 13... focusing optical system, 14... deflection optical system, 15... support tube, 16... jacket tube, 17... buffer fluid, 30... measurement unit, 31... light source, 32... optical branching unit, 33... optical detector, 34... optical terminal, 35... reflecting mirror, 36... analyzer, 37... output port.

## Claims

1. An optical probe comprising:
an optical fiber for transmitting light between a proximal end and a distal end;
an optical connecter being connected to the optical fiber at the proximal end;
a focusing optical system being connected to the optical fiber at the distal end and focusing light emitted from the distal end of the optical fiber;
a deflection optical system being connected to the optical fiber at the distal end and deflecting light emitted from the distal end of the optical fiber;
a jacket tube surrounding the optical fiber to extend along the optical fiber, and being rotatable relative to the optical fiber, the optical connecter, the focusing optical system, and the deflection optical system; and
a buffer fluid filled in an inner lumen of the jacket tube,
wherein the optical fiber has a cutoff wavelength shorter than 1.53 µm, and
the optical fiber, the focusing optical system, the deflection optical system, and the buffer fluid and jacket tube on an optical path coupled to a fundamental mode of the optical fiber have a light transmittance of -2 dB to 0 dB in a wavelength band of 1.6 µm to 1.8 µm.

2. The optical probe according to claim 1, wherein
each of the optical fiber, the focusing optical system, and the deflection optical system comprises either silica glass or borosilicate glass,
the buffer fluid is any one of a physiological saline solution, a dextran solution, and a silicone oil,
the jacket tube comprises any one of FEP, PFA, PTFE, PET, and nylon, and
a relative refractive index difference at one of an interface between the deflection optical system and the buffer fluid and an interface between the buffer fluid and the jacket tube differs from an relative refractive index difference at the other interface, by 3.2 times or more.

3. An optical measurement method through the use of the optical probe according to claim 1, a light source generating light in a wavelength band of 1.6 µm to 1.8 µm, an optical branching unit branching light emitted from the light source into two and outputting the resulting light as illumination light and reference light, an optical detector detecting light in the wavelength band, and an analyzer analyzing a light attenuation spectrum in the wavelength band and acquiring an analysis result obtained by the analysis, as image information, the method comprising the steps of:
irradiating an object with illumination light, the illumination light output from the optical branching unit to enter the proximal end of the optical fiber and to be emitted from the distal end;
guiding back-reflection light to the optical detector, the back-reflection light generated by the object along with the irradiation, to enter the distal end of the optical fiber and to be emitted from the proximal end, while guiding the reference light output from the optical branching unit to the optical detector;
detecting, with the optical detector, interference light caused by the back-reflection light and the reference light; and
analyzing a spectrum of the back-reflection light with the analyzer, and acquiring distribution information of a substance inside the object as image information.

4. The optical measurement method according to claim 3, wherein
each of the optical fiber, the focusing optical system, and the deflection optical system comprises either silica glass or borosilicate glass,
the buffer fluid is any one of a physiological saline solution, a dextran solution, and a silicone oil,
the jacket tube comprises any one of FEP, PFA, PTFE, PET, and nylon, and
a relative refractive index difference at one of an interface between the deflection optical system and the buffer fluid and an interface between the buffer fluid and the jacket tube differs from a relative refractive index difference at the other interface, by 3.2 times or more.

5. The optical measurement method according to claim 3, further comprising the steps of:
extracting a spectral component having an absorption peak in a wavelength range of 1.70 to 1.75 µm in a spectrum of the back-reflection light, with the analyzer; and
analyzing distribution information of lipid on the basis of the spectral component, and acquiring the analysis result as image information.

6. The optical measurement method according to claim 4, further comprising the steps of:
detecting, with the optical detector, interference light caused by reflected light and the reference light, the reflected light caused by reflection of the illumination light output from the optical branching unit by the one of the interfaces and reaching the optical detector after the reflection;
conducting Fourier analysis of a spectrum of the reflected light in a limited wavelength band and calculating an autocorrelation function as a function of delay time, with the analyzer; and
calculating wavelength dependency that a delay time bringing the value of this autocorrelation function to a peak has among the wavelength bands, and calculating an estimated value of wavelength dispersion of the back-reflection light.
